# EUROPEAN PATENT SPECIFICATION

(11) **EP 0 810 223 B1**
(45) Date of publication and mention of the grant of the patent: **25.07.2001**
(21) Application number: 97303343.4
(22) Date of filing: 16.05.1997
(51) Int. Cl.: C07D 401/14, C07D 401/06, A61K 31/44

(54) **Adamantyl substituted oxindoles as pharmaceutical agents**
Adamantyl substituierte Oxindole als pharmazeutische Wirkstoffe
Oxindoles substituées par adamantyle, comme agents pharmaceutiques

(30) Priority: 28.05.1996 US 18490 P
(43) Date of publication of application: 03.12.1997
(73) Proprietor: PFIZER INC., New York, N.Y. 10017 (US)
(72) Inventor: Lyssikatos, Joseph Peter, Gales Ferry, Connecticut 06335 (US); Volkmann, Robert Alfred, Mystic, Connecticut 06355 (US)
(74) Representative: Eddowes, Simon

(56) References cited:
- EP-A- 0 760 239
- WO-A-93/14085
- WO-A-93/23375
- WO-A-95/32949
- US-A- 4 876 259
- US-A- 5 258 401

## Description

This invention relates to the use of certain substituted heterocyclic compounds for the treatment of cancer. The therapeutically active agents of this invention exhibit activity as inhibitors of the enzyme farnesyl protein transferase and are believed to be useful as anti-cancer and anti-tumor agents.

Other compounds that inhibit farnesyl protein transferase and are believed to be useful as anti-cancer and anti-tumor agents are referred to in International Patent Application PCT/US 92/11292, which designates the United States and was published on July 22, 1993 as WO 93/14085, United States Patent 4,876,259, which issued on October 24, 1989, International Patent Application PCT/IB95/00189 filed on March 20, 1995 and published on November 9, 1995 as WO 95/29909, and United States Patent Application 08/737,376 filed on March 20, 1995 and issued on December 29, 1998 as United States Patent No. 5,854,232.

WO 93/23375 describes pyridylmethoxycarbonyl adamantyl derivatives and their use for the treatment of androgen dependent cancers. These compounds are not structurally close to the compounds claimed in the present invention. WO 95/32949 describes inhibitors of farnesylation of the "Ras" oncogene protein which are useful for the treatment of cancer. These compounds have a structure based on hexahydrooxepine and, again, are not structurally close to the presently claimed compounds.

Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer.

To acquire transforming potential, the precursor of the Ras oncoprotein must undergo farnesylation of the cysteine residue located in a carboxyl-terminal tetrapeptide. Inhibitors of the enzyme that catalyzes this modification, farnesyl protein transferase, have therefore been suggested as anticancer agents for tumors in which Ras contributes to transformation. Mutated, oncogenic forms of Ras are frequently found in many human cancers, most notably in more than 50% of colon and pancreatic carcinomas (Kohl et al., Science, Vol. 260, 1834 to 1837, 1993).

### Summary of the Invention

This invention relates to compounds of the formula 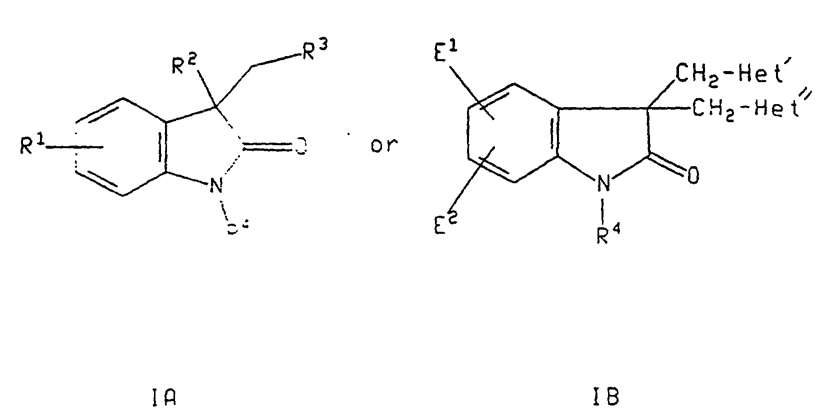 wherein
R¹ is hydrogen, chloro, fluoro, bromo or iodo, cyano, hydroxy, nitro, trifluoromethyl, -NHR⁵, -NR⁵R⁵, R⁵, -OR⁵ or -S(O)ₘ-R⁵;
R² is -(CH₂)ₙ-Y or -OCOR⁵;
R³ is 4-, 3-, or 2-pyridyl, pyrimidyl, pyrazinyl, 2-fluoro-4-pyridyl or 3-fluoro-4-pyridyl;
R⁴ is 1-adamantyl or 2-adamantyl;
Y is hydrogen, hydroxy, amino, cyano, -NHR⁵, -NR⁵R⁵, -NHCOR⁵, -NHCO₂R⁵, halo, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, phenyl, phenyl substituted with W, -C≡CCO₂R⁵, -CH=CHR⁵ or -C≡CR⁵;
each R⁵ is, independently, (C₁-C₄) straight or branched alkyl, phenyl or benzyl, wherein said phenyl and the phenyl moiety of said benzyl may optionally be substituted with halo, hydroxy, nitro, cyano, amino, (C₁-C₄) straight or branched alkyl, (C₁-C₄) straight or branched alkoxy, phenyl, benzyl, (C₁-C₄)alkylamino, di[(C₁-C₄)alkyl]amino, or -S(O)ₘ-(C₁-C₄) straight or branched alkyl;
each W is, independently, halo, R⁵, hydroxy, -OR⁵, nitro, amino, -NHR⁵, -NR⁵R⁵, cyano, or -S(O)ₘ-R⁵;
m is 0, 1 or 2;
n is 1 to 7;
E¹ and E² are selected, independently, from hydrogen, halo, (C₁-C₃)alkyl, hydroxy, (C₁-C₃)alkoxy, nitro, trifluoromethyl, cyano, amino, (C₁-C₃)alkylamino and di[(C₁-C₃)alkyl]amino;
Het' and Het" are independently selected from the group consisting of 6 membered heterocyclic rings comprising a 6-membered carbocyclic ring wherein from one to four carbon atoms thereof is each replaced by a nitrogen atom, optionally substituted with one substituent selected from (C₁-C₃) alkyl, halo, hydroxy, (C₁-C₃)alkoxy, amino, (C₁-C₃)alkylamino and di[(C₁-C₃)alkyl]amino;
and their pharmaceutically acceptable salts.

More specific embodiments of this invention include the following:
(a) compounds of the formula IA wherein R³ is 4-pyridyl, 4-pyrimidyl or 2-fluoro-4-pyridyl;
(b) compounds of the formula IA wherein R² is -(CH₂)ₙY;
(c) compounds of the formula IA wherein R² is -(CH₂)ₙY and n is an integer from 1 to 5;
(d) compounds of the formula IA or IB wherein each of R¹, E¹ and E² is hydrogen; and
(e) compounds of the formula IA wherein R² is -(CH₂)ₙ-Y, R¹ is 4-pyridyl, 4-pyrimidyl or 2-fluoro-4-pyridyl, R⁵ is (C₁-C₂) alkyl and Y is -CO₂R⁵, cyano, -CONHR⁵, -CH=CHCO₂R⁵ or -OCOR⁵.

Other more specific embodiments of this invention relate to compounds of the formula IA wherein the R⁵ groups are other than a phenyl or benzyl group that is substituted with either a phenyl or benzyl group. Other more specific embodiments of this invention relate to compounds of the formula IA wherein the R⁵ groups are other than substituted or unsubstituted phenyl or benzyl.

This invention also relates to a method of inhibiting the abnormal growth of cells in a mammal, including a human, comprising administering to said mammal a farnesyl protein transferase inhibiting effective amount of a compound of the formula IA or-IB, as defined above, or a pharmaceutically acceptable salt of such a compound.

This invention also relates to a method of inhibiting the abnormal growth of cells in a mammal, including a human, comprising administering to said mammal an abnormal cell growth inhibiting effective amount of a compound of the formula IA or IB, as defined above, or a pharmaceutically acceptable salt of such a compound.

This invention also relates to a pharmaceutical composition for inhibiting the abnormal growth of cells in a mammal, including a human, comprising a farnesyl protein transferase inhibiting effective amount of a compound of the formula IA or IB, as defined above, or a pharmaceutically acceptable salt of such a compound, and a pharmaceutically acceptable carrier.

This invention also relates to a pharmaceutical composition for inhibiting the abnormal growth of cells in a mammal, including a human, comprising and administering to said mammal and abnormal cell growth inhibiting effective amount of a compound of the formula IA or IB, as defined above, or pharmaceutically acceptable salt of such a compound, and pharmaceutically acceptable carrier.

"Abnormal cell growth", as used herein, refers to cell growth that is independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

Examples of such benign proliferative diseases are psoriasis, benign prostatic hypertrophy and restinosis.

The term "alkyl", as used herein, unless otherwise indicated, includes saturated monovalent hydrocarbon radicals having straight, branched or cyclic moieties or combinations thereof.

The term "halo", as used herein, refers to chloro, fluoro, bromo or iodo.

The compounds of formulae IA and IB that are basic in nature are capable of forming a wide variety of salts with various inorganic and organic acids. The acids that may be used to prepare pharmaceutically acceptable acid addition salts of those compounds of formulae IA and IB that are basic in nature are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts.

The compounds of formulae IA and IB above may contain chiral centers and therefore may exist in different enantiomeric forms. This invention relates to all the optical isomers and other stereoisomers of compounds of the formulae IA and IB, as well as racemic and other mixtures of such isomers,

Patients that can be treated with compounds of the formula IA or IB according to the methods of this invention include, for example, patients that have been diagnosed as having lung cancer, bone cancer, pancreatic cancer, skin cancer, cancer of the head and neck, cutaneous or intraocular melanoma, uterine cancer, ovarian cancer, rectal cancer, cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's Disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphonas, cancer of the bladder, cancer of the kidney or ureter (e.g., renal cell carcinoma, carcinoma of the renal pelvis), or neoplasms of the central nervous system (e.g., primary CNS lymphona, spinal axis tumors, brain stem gliomas or pituitary adenomas).

Patients that can be treated with compounds of the formula IA or IB according to the methods of this invention also include patients suffering from abnormal cell growth, as defined above.

### Detailed Description Of The Invention

The preparation of compounds of the formulae IA and IB are described below. In the reaction schemes and discussion that follow, Y, W, R¹, R², R³, R⁴, R⁵, E¹, E², Het' and Het" are defined as above.

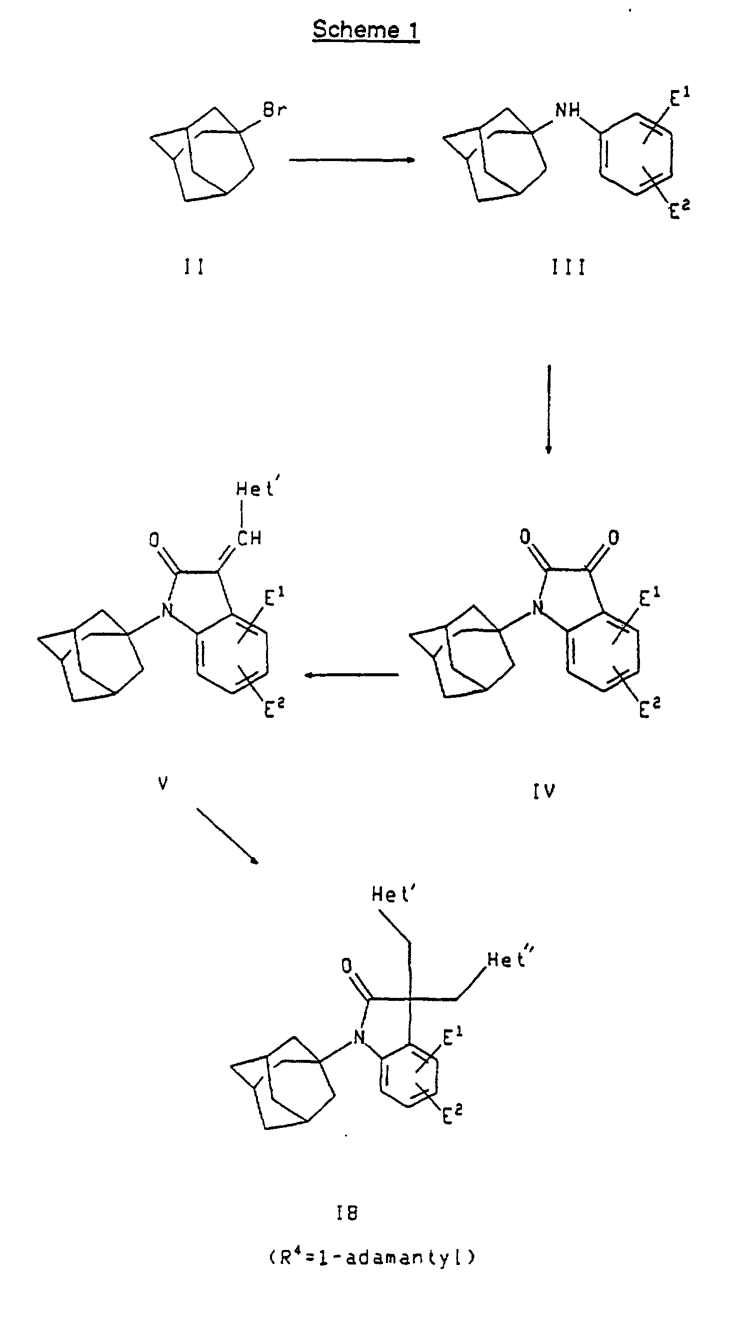

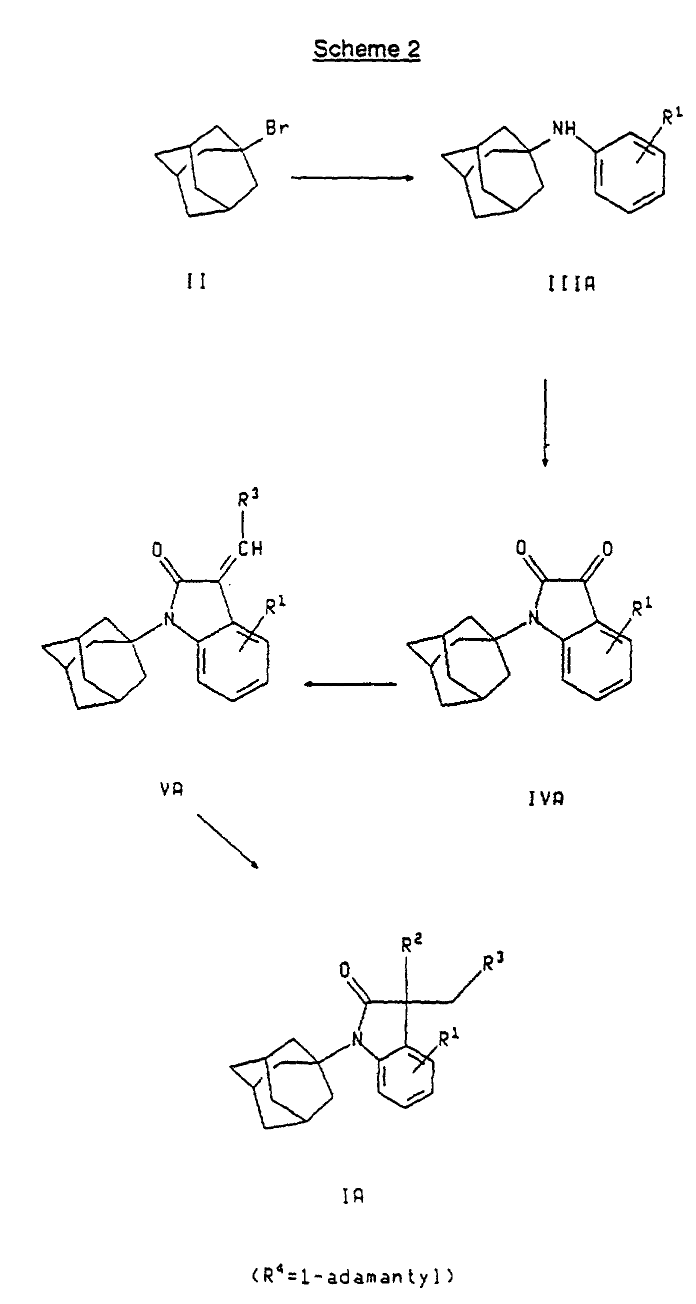

Scheme 1 illustrates the synthesis of compounds of the formula IB wherein R⁴ = 1-adamantyl. Compounds of the formula IB wherein R⁴ is 2-adamantyl can be prepared by the same process starting with the 2-adamantyl analog of compound II.

Referring to Scheme 1,1-bromoadamantyl (formula II) is reacted with aniline or an aniline derivative of the formula 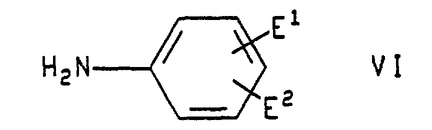 to form the adamantyl substituted aniline derivative of formula III. This reaction is generally carried out neat at a temperature from about 150°C to about 250°C, preferably at about 200°C. The compound of formula III is then reacted with oxalyl chloride to form the substituted benzopyrrolidine of formula IV. Typically, this reaction is carried out in an aprotic solvent such as benzene or toluene, preferably toluene, at a temperature from about 0°C to about 80°C, preferably at about 65°C.

Reaction of the resulting compound of formula IV with a compound of the formula CH₃-Het' in an acetic acid/acetic anhidride mixture yields the corresponding compound of formula V. The temperature for this reaction can range from about 100° C to about 160°C, and is preferably about 140°C.

The compound of formula V so formed can be converted into the desired compound of formula IB by the following two step process. First, the compound of formula V is reacted with a reducing agent such as sodium triacetoxy borohydride or sodium borohydride in methanol or ethanol, preferably sodium borohydride in methanol, at a temperature of about 0°C to about 30°C, preferably at about 5°C, to reduce the carbon-carbon double bond of the Het'-CH= sidechain. Then, the Het"-CH₂ substituent is added in situ, or after isolating the product of the foregoing reaction, by reacting the foregoing reaction mixture, or the isolated product, as the case may be, with Het"-CH₂X, wherein X is an appropriate leaving group such as chloro or bromo, in the presence of a strong base such as potassium hydroxide in methanol or sodium hydride in either tetrahydrofuran (THF), ether or dimethoxy ethane (DME), preferably potassium hydroxide in methanol or sodium hydride in THF. This reaction is typically carried out at a temperature from about 0°C to about 60°C. Preferably the reaction temperature is from about 20°C to about 30°C. If the reaction with Het-CH₂X is carried out in situ, it is helpful to add potassium hybroxide to the mixture as a solubilizer.

Scheme 2 illustrates the synthesis of compounds of the formula IA wherein R⁴ is 1-adamantyl. The corresponding compounds wherein R⁴ is 2-adamantyl can be prepared in the same manner starting with the 2-adamantyl analog of compound II.

Referring to Scheme 2, the compounds having formulae IIIA, IVA and VA can be prepared as described above for the formation of compounds having the formulae III, IV and V, respectively, with the exception that the reagent of formula VI is replaced with a compound of the formula 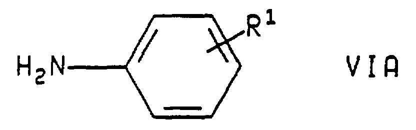 and the reagent of formula methyl-Het' is replaced with a compound of the formula CH₃R³.

The desired product of formula IA can then be formed as follows. The compound of formula VA is first reacted with potassium bis(trimethylsilyl)amide in THF at a temperature from about -70°C to about 60°C, preferably at about 0°C. Then, after stirring for about 30 minutes, a compound of formula R²X, wherein X is an appropriate leaving group (e.g., chloride or bromide), is added and the reaction mixture is allowed to warm to about ambient temperature.

World Patent Application WO 93/14085, referred to above and incorporated herein by reference in its entirety, describes a method of preparing compounds that differ from those of the formula IA in that there is no adamantyl substituent on the ring nitrogen.

United States Patent Application 4,876,259, also referred to above and incorporated herein by reference in its entirety, describes methods of synthesizing compounds that differ from those of the formula IB in that there is no adamantyl substituent on the ring nitrogen.

The starting materials used in the processes of Schemes 1 and 2 are either known in the literature or commercially available.

The compounds of the formulae IA and IB that are basic in nature are capable of forming a wide vanety of different salts with various inorganic and organic acids. Although such salts must be pharmaceutically acceptable for administration to animals, it is often desirable in practice to initially isolate the compound of formula I, IA and IB from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

The compounds of the formulae IA and IB exhibit activity as Ras farnesylation inhibitors and are useful in the treatment of cancer and the inhibition of abnormal cell growth in mammals, including humans.

Patients that can be treated with compounds of the formula IA or IB according to the methods of this invention include, for example, patients that have been diagnosed as having lung cancer, bone cancer, pancreatic cancer, skin cancer, cutaneous or intraocular melanoma, uterine cancer, ovarian cancer, rectal cancer, cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's Disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostrate cancer, chronic or acute leukemia, solid tumors of childhood, lymphocytic lymphonas, cancer of the bladder, cancer of the kidney or ureter (e.g., renal cell carcinoma, carcinoma of the renal pelvis), or neoplasms of the central nervous system (e.g., primary CNS lymphona, spinal axis tumors, brain stem gliomas or pituitary adenomas).

The compounds of formulae IA and IB and their pharmaceutically acceptable salts (hereinafter referred to, collectively, as "the therapeutic compounds") can be administered orally, transdermally (e.g., through the use of a patch), parenterally or topically. Oral administration is preferred. In general, compounds of the formula I and their pharmaceutically acceptable salts are most desirably administered in dosages ranging from about 1.0 mg up to about 500 mg per day, preferably from about 1 to about 100 mg per day in single or divided (i.e., multiple) doses. Compounds of the formulae IA and IB and their pharmaceutically acceptable salts will ordinarily be administered in daily dosages ranging from about 0.01 to about 10 mg per kg body weight per day, in single or divided doses. Variations may occur depending on the weight and condition of the person being treated and the particular route of administration chosen. In some instances, dosage levels below the lower limit of the aforesaid range may be more than adequate, while in other cases still larger doses may be employed without causing any harmful side effect, provided that such larger doses are first divided into several small doses for administration throughout the day.

The therapeutic compounds may be administered alone or in combination with pharmaceutically acceptable carriers or diluents by either of the two routes previously indicated, and such administration may be carried out in single or multiple doses. More particularly, the novel therapeutic compounds of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, elixirs, syrups, and the like. Such carriers include solid diluents or fillers, sterile aqueous media and various non-toxic organic solvents, etc. Moreover, oral pharmaceutical compositions can be suitably sweetened and/orflavored.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic compound in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well-known to those skilled in the art.

Additionally, it is also possible to administer the therapeutic compounds topically and this may preferably be done by way of creams, jellies, gels, pastes, ointments and the like, in accordance with standard pharmaceutical practice.

The activity of the therapeutic compounds as ras farnesylation inhibitors may be determined by their ability, relative to a control, to inhibit ras farnesyl transferase in vitro. This procedure is described below.

A crude preparation of human famesyl transferase (FTase) comprising the cytosolic fraction of homogenized brain tissue is used for screening compounds in a 96-well assay format. The cytosolic fraction is prepared by homogenizing approx. 40 grams fresh tissue in 100 ml of sucrose/MgCl₂/EDTA buffer (using a Dounce homogenizer; 10-15 strokes), centrifuging the homogenates at 1000 grams for 10 minutes at 4G, re-centrifuging the supernatant at 17,000 grams for 15 minutes at 4G, and then collecting the resulting supernatant. This supernatant is diluted to contain a final concentration of 50 mM Tris HCI (pH 7.5), 5 mN DTT, 0.2 M KCl, 20 *µ*M ZnCl₂, 1 mM PMSF and re-centrifuged at 178,000 grams for 90 minutes at 4G. The supernatant, termed "crude FTase" was assayed for protein concentration, aliquoted, and stored at -70°C.

The assay used to measure in vitro inhibition of human FTase is a modification of the method described by Amersham LifeScience for using their Farnesyl transferase (3H) Scintillation Proximity Assay (SPA) kit (TRKQ 7010). FTase enzyme activity is determined in a volume of 100 *µ*l containing 50 mM N-(2-hydroxy ethyl) piperazine-N'-(2-ethane sulfonic acid) (HEPES), pH 7.5, 30 mM MgCl₂, 20 uM KCI, 5 mM Na₂HPO₄, 5 mM dithiothreitol (DTT), 0.01% Triton X-100, 5% dimethyl sulfoxide (DMSO), 20 *µ*g of crude FTase, 0.12 *µ*M [3H]-farnesyl pyrophosphate ([3H]-FPP; 36000 dpm/pmole, Amersham LifeScience), and 0.2 *µ*M of biotinylated Ras peptide KTKCVIS (Bt-KTKCVIS) that is N-terminally biotinylated at its alpha amino group and was synthesized and purified by HPLC in house. The reaction is initiated by addition of the enzyme and terminated by addition of EDTA (supplied as the STOP reagent in kit TRKQ 7010) following a 45 minute incubation at 37°C. Prenylated and unprenylated Bt-KTKCVIS is captured by adding 10 *µ*l of steptavidin-coated SPA beads (TRKQ 7010) per well and incubating the reaction mixture for 30 minutes at room temperature. The amount of radioactivity bound to the SPA beads is determined using a MicroBeta 1450 plate counter. Under these assay conditions, the enzyme activity is linear with respect to the concentrations of the prenyl group acceptor, Bt-KTKCVIS, and crude FTase, but saturating with respect to the prenyl donor, FPP. The assay reaction time is also in the linear range.

The test compounds are routinely dissolved in 100% DMSO. Inhibition of farnesyl transferase activity is determined by calculating percent incorporation of tritiated-farnesyl in the presence of the test compound vs. its incorporation in control wells (absence of inhibitor). IC₅₀ values, that is, the concentration required to produce half maximal farnesylation of Bt-KTKCVIS, is determined from the dose-responses obtained.

### EXAMPLE 1

### 1-Adamantyl-1-yl-3,3-bis-pyridin-4-ylmethyl-1,3-dihydro-indol-2-one

### A. N-1-Adamantylaniline

Under a nitrogen (N₂) atmosphere was combined 10.0 g (46.5 mmol) of 1-bromoadamantane and 20 ml of aniline. The reaction was stirred for 20 hours at 200°C, and then cooled and fractionated on silica gel using 6:1 hexane: ethyl acetate (EtOAc) to afford, after concentration in vacuo and refractionation using toluene, 5.65 g (54%) of N-1-adamantylaniline.
¹H NMR (CDCl₃) 1.60-1.70 (m-6H), 1.80-1.90 (m-6H), 2.05-2.15 (m-3H), 3.00-3.40 (bs-1H), 6.70-6.80 (m-3H), 7.10-7.20 (m-2H).
¹³C NMR (CDCl₃) 29.64, 36.38, 43.37, 52,16, 119.02, 199.08, 128.62, 145.86.

### B. 1-Adamantylisatin

Under a N₂ atmosphere was added 1.97 g (15.6 mmol) of oxalyl chloride to 3 ml of dry toluene which was cooled to 0°C. To this solution was added 3.55g (15.6 mmol) of N-1-adamantylaniline in toluene (8 ml). The reaction was allowed to stir for 30 min. at 0°C and then heated at 65°C for 3 hours. Additional solvent (10 ml) was added and the reaction was kept at 65°C for 72 hours. The solvent was removed and the residue was allowed to stir at 160°C for 5 hours. The crude reaction mixture was allowed to cool and was chromatographed on silica gel using 6:1 hexane: EtOAc to afford crude product, which was triturated with isopropyl ether (IPE) to generate 164 mg (4.4%) of 1-adamantylisatin combined with product contaminated with significant amounts of impurities.
¹H NMR (CDCl₃) 1.70-1.80 (m-6H), 2.20-2.25 (m-3H), 2.50-2.60 (m-6H). 7.00-7.70 (m-5H).
¹³C NMR (CDCl₃) 29.78, 36.14, 40.07, 61.26, 115.62, 118.96, 122.87, 125.51, 137.42, 152.15.

### C. 1-Adamantyl-1-yl-3-pyridin-4-ylmethyl-1,3-dihydro-indol-2-one

Under a N₂ atmosphere was added 164 mg (0.567 mmol) of 1-adamantylisatin to 2 ml of glacial acetic acid. The suspension was warmed in an oil bath. 4-Picoline (0.091 ml, 0.94 mmol) followed by acetic anhydride (0.094 ml, 1.00 mmol) was added and the solution was allowed to stir at 140°C for 18 hours. The reaction mixture was cooled and quenched with water. ETOAc was added and the aqueous layer was made basic with sodium bicarbonate (NaHCO₃). The organic layer was washed with water followed by brine and then dried over magnesium sulfate, filtered and concentrated in vacuo to afford crude product, which was purified on silica gel using 1:1 ETOAc: hexane to afford 54 mg of starting 1-adamantylisatin and 108 mg (52%) of the desired 1 -adamantyl-1 -yl-3-pyridin-4-ylmethyl-1,3-dihydro-indol-2-one as a mixture of geometric isomers.
¹H NMR (CDCl₃) 1.65-1.85 (m-6H), 2.20-2.35 (m-3H), 2.50-2.62 (m-6H), 6.70-7.90 (m-9H), 8.70-8.82 (m-1H).
¹³C NMR (CDCl₃) 29.70, 36.09, 39.99, 60.32, 113.75, 113.97, 119.58, 120.94, 121.46, 122.81, 122.87, 124.76, 129.46, 129.63, 131.06, 143.60, 148.79, 150.00.

### D. 1-Adamantyl-1-yl-3,3-bis-pyridin-4-ylmethyl-1,3-dihydro-indol-2-one

To a methanol (8 ml) solution at 0-5°C under a N₂ atmosphere containing 1-adamantyl-1-yl-3-pyridin-4-ylmethyl-1,3-dihydro-indol-2-one (100 mg, 0.28 mmol) was added 17 mg (0.45 mg) of sodium borohydride (NaBH₄). The reaction mixture was allowed to stir for 45 minutes, at which time 1 ml of water (H₂O) followed by 73 mg (1.12 mmol) of potassium hydroxide (KOH) in H₂O was added. After 2-3 minutes, 4-picolyl chloride-hydrochlonde (49.5 mg, 3 mmol) was added and the reaction was allowed to stir for an additional 15 minutes. Tetrahydrofuran (THF) (2 ml) was added to help solubilize the reactants. After 1 hour, the reaction mixture was concentrated to approximately 5 ml, additional THF was added and the reaction was allowed to stir for 16 hours. The reaction mixture was then concentrated in vacuo and taken up in EtOAc (50 mls). The organic extract was washed with water (3X) and then brine, and then dried over magnesium sulfate and concentrated in vacuo and purified on silica gel using EtOAc to afford 47 mg (38%) of the desired 1-adamantyl-1-yl-3,3-bis-pyridin-4-ylmethyl-1,3-dihydro-indol-2-one.
¹H NMR (CDCl₃) 1.58-1.62 (m-6H), 1.95-2.10 (m-9H), 3.14 (q-4H: JAB=12.5 Hz), 6.70-8.40 (m-8H), 8.20-8.32 (m-4H).
¹³C NMR (CDCl₃) 29.70, 36.09, 39.99, 60.32, 113.75, 113.97, 119.58, 120.94.

## Claims

1. A compound of the formula 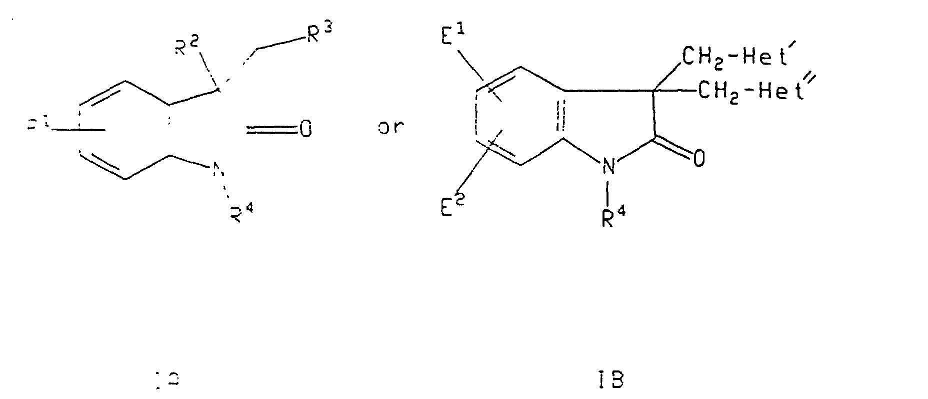 wherein
R¹ is hydrogen, chloro, fluoro, bromo or iodo, cyano, hydroxy, nitro, trifluoromethyl, -NHR⁵, -NR⁵R⁵, R⁵, -OR⁵ or -S(O)ₘ-R⁵;
R² is -(CH₂)ₙ-Y or -OCOR⁵;
R³ is 4-, 3-, or 2-pyridyl, pyrimidyl, pyrazinyl, 2-fluoro-4-pyridyl or 3-fluoro-4-pyridyl;
R⁴ is 1-adamantyl or 2-adamantyl;
Y is hydrogen, hydroxy, amino, cyano, -NHR⁵, -NR⁵R⁵, -NHCOR⁵, -NHCO₂R⁵, halo, OR⁵, -S(O)ₙR⁵, -CO₂H, -CO₂R⁵, -CONR⁵R⁵, -CONHR⁵, -CONH₂, -COR⁵, -CH=CHCO₂R⁵, -OCOR⁵, phenyl, phenyl substituted with W, -C≡CCO₂R⁵, -CH=CHR⁵ or -C≡CR⁵;
each R⁵ is. independently, (C₁-C₄) straight or branched alkyl, phenyl or benzyl, wherein said phenyl and the phenyl moiety of said benzyl may optionally be substituted with halo, hydroxy, nitro, cyano, amino, (C₁-C₄) straight or branched alkyl, (C₁-C₄) straight or branched alkoxy, phenyl, benzyl, (C₁-C₄)alkylamino, di[(C₁-C₄)alkyl]amino, or -S(O)ₘ-(C₁-C₄) straight or branched alkyl;
each W is, independently, halo, R⁵, hydroxy, -OR⁵, nitro, amino, -NHR⁵, -NR⁵R⁵, cyano, or -S(O)ₘ-R⁵.
m is 0, or 2;
n is 1 to 7;
E¹ and E² are selected, independently, from hydrogen, halo, (C₁-C₃)alkyl, hydroxy, (C₁-C₃)alkoxy, nitro, trifluoromethyl, cyano, amino, (C₁-C₃)alkylamino and di[(C₁-C₃)alkyl]amino;
Het' and Het" are independently selected from the group consisting of 6 membered heterocyclic rings comprising a 6-membered carbocyclic ring where from one to four carbon atoms thereof is each replaced by a nitrogen atom, optionally substituted with one substituent selected from (C₁-C₃) alkyl, halo, hydroxy, (C₁-C₃) alkoxy, amino, (C₁-C₃) alkylamino and di[(C₁-C₃)alkyl]amino;
or a pharmaceutically acceptable salt thereof.

2. The compound 1-adamantyl-1-yl-3,3-bis-pyridin-4ylmethyl-1,3-dihydro-indol-2-one.

3. A pharmaceutical composition for inhibiting abnormal cell growth in a mammal comprising a compound according to claim 1 or claim 2 and a pharmaceutically acceptable carrier.

4. The use of a compound as claimed in claim 1 for the preparation of a medicament for treating cancer in a mammal.

5. The use according to claim 4 wherein said medicament is intended for the treatment of breast cancer, bone cancer, lung cancer, pancreatic cancer, skin cancer, prostate cancer or colon cancer.

## Patentansprüche

1. Verbindung der Formel 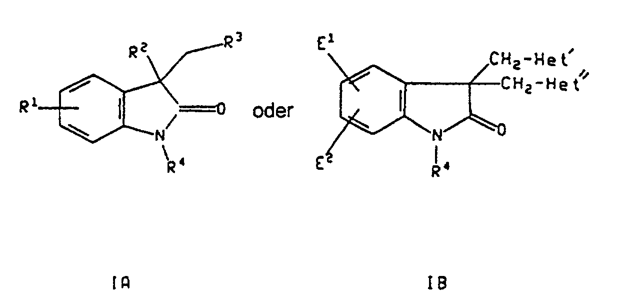 worin
R¹ Wasserstoff, Chlor, Fluor, Brom oder Jod, Cyano, Hydroxy, Nitro, Trifluormethyl, -NHR⁵, -NR⁵R⁵, R⁵, -OR⁵ oder -S(O)ₘ-R⁵ darstellt;
R² -(CH₂)ₙ-Y oder -OCOR⁵ darstellt;
R³ 4-, 3- oder 2-Pyridyl, Pyrimidyl, Pyrazinyl, 2-Fluor-4-pyridyl oder 3-Fluor-4-pyridyl darstellt;
R⁴ 1-Adamantyl oder 2-Adamantyl darstellt;
Y Waserstoff, Hydroxy, Amino, Cyano, -NHR⁵, -NR⁵R⁵, -NHCOR⁵, -NHCO₂R⁵, Halogen, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, Phenyl, Phenyl, substituiert mit W, -C≡CCO₂R⁵, -CH=CHR⁵ oder -C≡CR⁵, darstellt;
wobei jeder Rest R⁵ unabhängig voneinander gerad- oder verzweigtkettiges (C₁-C₄)-Alkyl, Phenyl oder Benzyl darstellt, wobei das Phenyl und die Phenyleinheit des Benzyls gegebenenfalls mit Halogen, Hydroxy, Nitro, Cyano, Amino, gerad- oder verzweigtkettigem (C₁-C₄)-Alkyl, gerad- oder verzweigtkettigem (C₁-C₄)-Alkoxy, Phenyl, Benzyl, (C₁-C₄)-Alkylamino, Di [(C₁-C₄)-alkyl]amino oder gerad- oder verzweigtkettigem -S(O)ₘ-(C₁-C₄)-Alkyl substituiert sein können;
wobei W unabhängig voneinander Halogen, R⁵, Hydroxy, -OR⁵, Nitro, Amino, -NHR⁵, -NR⁵R⁵, Cyano oder -S(O)ₘ-R⁵ darstellt;
m 0, 1 oder 2 ist;
n 1 bis 7 ist;
E¹ und E² unabhängig voneinander aus Wasserstoff, Halogen, (C₁-C₃)-Alkyl, Hydroxy, (C₁-C₃)-Alkoxy, Nitro, Trifluormethyl, Cyano, Amino, (C₁-C₃)-Alkylamino und Di[(C₁-C₃)alkyl]amino ausgewählt sind;
Het' und Het" unabhängig voneinander aus der Gruppe, bestehend aus 6-gliedrigen, heterocyclischen Ringen, umfassend einen 6-gliedrigen carbocyclischen Ring, worin ein bis vier Kohlenstoffatome davon jeweils durch ein gegebenenfalls mit einem Substituenten, ausgewählt aus (C₁-C₃)-Alkyl, Halogen, Hydroxy, (C₁-C₃)-Alkoxy, Amino, (C₁-C₃)-Alkylamino und Di[(C₁-C₃)alkyl]amino, substituiertes Stickstoffatom ersetzt sind, ausgewählt sind;
oder ein pharmazeutisch verträgliches Salz davon.

2. Verbindung 1-Adamantyl-1-yl-3,3-bis-pyridin-4-ylmethyl-1,3-dihydro-indol-2-on.

3. Pharmazeutische Zusammensetzung zur Inhibierung von anormalem Zellwachstum in einem Säuger, umfassend eine Verbindung nach Anspruch 1 oder Anspruch 2, und einen pharmazeutisch verträglichen Träger.

4. Verwendung einer Verbindung nach Anspruch 1 zur Herstellung eines Arzneimittels zur Behandlung von Krebs in einem Säuger.

5. Verwendung nach Anspruch 4, wobei das Arzneimittel zur Behandlung von Brustkrebs, Knochenkrebs, Lungenkrebs, Pankreaskrebs, Hautkrebs, Prostatakrebs oder Colonkrebs vorgesehen ist.

## Revendications

1. Composé de formule générale 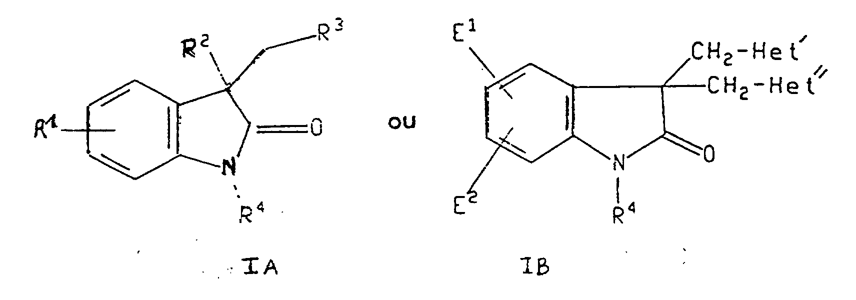 dans laquelle
R¹ représente l'hydrogène, un groupe chloro, fluoro, bromo ou iodo, cyano, hydroxy, nitro, trifluorométhyle, -NHR⁵, -NR⁵R⁵, R⁵, -OR⁵ ou -S(O)ₘ-R⁵ ;
R² représente un groupe -(CH₂)ₙ-Y ou -OCOR⁵ ;
R³ représente un groupe 4-, 3- ou 2-pyridyle, pyrimidyle, pyrazinyle, 2-fluoro-4-pyridyle ou 3-fluoro-4-pyridyle ;
R⁴ représente un groupe 1-adamantyle ou 2-adamantyle ;
Y représente l'hydrogène, un groupe hydroxy, amino, cyano, -NHR⁵, -NR⁵R⁵, -NHCOR⁵, -NHCO₂R⁵, halogéno, OR⁵, -S(O)ₘR⁵, -CO₂H, -CO₂R⁵, -CONR⁵R⁵, -CONHR⁵, -CONH₂, -COR⁵, -CH=CHCO₂R⁵, -OCOR⁵, phényle, phényle substitué avec W, -C≡CCO₂R⁵, -CH=CHR⁵ ou -C≡CR⁵ ;
chaque groupe R⁵ représente, indépendamment, un groupe alkyle en C₁ à C₄ droit ou ramifié, phényle ou benzyle, ledit groupe phényle et le groupement phényle dudit groupe benzyle pouvant être facultativement substitués avec des substituants halogéno, hydroxy, nitro, cyano, amino, alkyle en C₁ à C₄ droit ou ramifié, alkoxy en C₁ à C₄ droit ou ramifié, phényle, benzyle, alkylamino en C₁ à C₄, di-(alkyle en C₁ à C₄) -amino ou -S(O)ₘ-(alkyle en C₁ à C₄) droit ou ramifié ;
chaque groupe W représente, indépendamment, un groupe halogéno, R⁵, hydroxy, -OR⁵, nitro, amino, -NHR⁵, -NR⁵R⁵, cyano ou -S(O)ₘ-R⁵ ;
m est égal à 0, 1 ou 2 ;
n a une valeur de 1 à 7 ;
E¹ et E² sont choisis, indépendamment, entre l'hydrogène, des groupes halogéno, alkyle en C₁ à C₃, hydroxy, alkoxy en C₁ à C₃, nitro, trifluorométhyle, cyano, amino, alkylamino en C₁ à C₃ et di-(alkyle en C₁ à C₃)-amino ;
Het' et Het" sont choisis indépendamment dans le groupe consistant en noyaux hétérocycliques hexagonaux comprenant un noyau carbocyclique hexagonal dans lequel 1 à 4 atomes de carbone de ce noyau sont remplacés chacun par un atome d'azote, facultativement substitué avec un substituant choisi entre des substituants alkyle en C₁ à C₃, halogéno, hydroxy, alkoxy en C₁ à C₃, amino, alkylamino en C₁ à C₃ et di-(alkyle en C₁ à C₃)-amino ;
ou ses sels pharmaceutiquement acceptables.

2. Composé consistant en 1-adamantyl-1-yl-3,3-bis-pyridine-4-ylméthyl-1,3-dihydro-indole-2-one.

3. Composition pharmaceutique pour inhiber une croissance cellulaire anormale chez un mammifère, comprenant un composé suivant la revendication 1 ou la revendication 2 et un support pharmaceutiquement acceptable.

4. Utilisation d'un composé suivant la revendication 1 pour la préparation des médicaments destinés au traitement du cancer chez un mammifère.

5. Utilisant suivant la revendication 4, dans laquelle le médicament est destiné au traitement du cancer du sein, du cancer des os, du cancer du poumon, du cancer du pancréas, du cancer de la peau, du cancer de la prostate ou du cancer du côlon.
